# EUROPEAN PATENT SPECIFICATION

(11) **EP 4 142 584 B1**
(45) Date of publication and mention of the grant of the patent: **16.10.2024**
(21) Application number: 21718899.4
(22) Date of filing: 20.04.2021
(51) Int. Cl.: A61B 5/024, A61B 5/0245, A61B 5/361, A61B 5/364

(54) **A METHOD FOR DETECTING AN ECTOPIC SIGNAL IN AN ELECTROCARDIOGRAM**
VERFAHREN ZUR DETEKTION EINES EKTOPISCHEN SIGNALS IN EINEM ELEKTROKARDIOGRAMM
PROCÉDÉ PERMETTANT DE DÉTECTER UN SIGNAL ECTOPIQUE DANS UN ÉLECTROCARDIOGRAMME

(30) Priority: 30.04.2020 US 202063017709 P; 01.07.2020 EP 20183409
(43) Date of publication of application: 08.03.2023
(73) Proprietor: BIOTRONIK SE & Co. KG, 12359 Berlin (DE)
(72) Inventor: GARNER, Garth, Tigard, OR 97224 (US); SHELLY, Iris, Portland, 97209 (US); WHITTINGTON, R. Hollis, Portland, OR 97202 (US)
(74) Representative: Biotronik Corporate Services SE
(86) International application number: PCT/EP2021/060174
(87) International publication number: WO 2021/219426

(56) References cited:
- US-B1- 10 398 381
- US-B2- 10 117 595
- US-B2- 7 171 260
- US-B2- 8 280 510
- US-B2- 8 897 863

## Description

The present invention relates in an aspect generally to a method for detecting an ectopic signal in an electrocardiogram. In another aspect, the present invention relates to an implantable medical device for detecting an electrical signal of a human or animal heart. In yet another aspect, the present invention relates to a method for detecting atrial fibrillation of a human or animal heart.

Implantable medical devices for detecting atrial fibrillation have to work reliably so as to be able to detect real atrial fibrillation but to not falsely detect atrial fibrillation. However, ectopic cardiac beats resulting in ectopic signals in an electrocardiogram (also referred to as ectopies) often distort atrial fibrillation detection. Thus, there is a need in reliably detecting ectopic events in an electrocardiogram.

Ectopies can be caused by premature atrial contractions (PACs) and by premature ventricular contractions (PVCs). PACs and PVCs are typically preceded with a short interval followed by a long compensatory pulse interval. PACs typically lead to the same QRS complex morphology like a regular cardiac rhythm. This is due to the fact that the electrical conduction to the ventricle makes use of the normal cardiac conduction pathway. In contrast, PVCs typically have a different ventricular morphology because of a different electrical conduction pathway. Algorithms known from prior art detecting ectopies (caused by PACs or PVCs) typically include interval timing looking for short-long intervals.

US8280510 B2 discloses determining ectopic intervals, which are then not used for calculating heart rate variability.

US 2013/0218037 A1 discloses a method for removal of ectopic beats by computing a ratio between an R-R interval comprising an ectopic signal and a preceding (presumably normal) R-R interval. Alternatively, an R-R interval comprising an ectopic beat is compared with a preceding and a succeeding R-R interval, wherein at the same time an R-R interval succeeding an R-R interval comprising an ectopic beat is compared to this R-R interval comprising an ectopic beat as well as to the next successive R-R interval.

US 8,977,350 B2 discloses a method for identifying ectopic beats by comparing R-R interval lengths of consecutive R-R intervals.

It is an object of the present invention to provide a method that allows detection of ectopic signals in an electrocardiogram more reliably than the methods known from prior art.

This object is achieved, in an aspect, by a method for detecting an ectopic signal in an electrocardiogram comprising the steps explained in the following. In this context, the ectopic signal is caused by an ectopic cardiac beat.

In a first step, consecutive R-R intervals are detected or identified in an electrocardiogram.

Afterwards, an average R-R interval is calculated for a determinable number of latest R-R intervals. To give an example, the determinable number of latest R-R intervals can be 1, 2, 3, 4, 5, 6, 7, 8, 9, or 10. According to an embodiment, the latest R-R intervals considered are the latest consecutive intervals. In an embodiment, the determinable number of latest consecutive R-R intervals is a number lying in a range between 2 and 20, in particular between 3 and 19, in particular between 4 and 18, in particular between 5 and 17, in particular between 6 and 16, in particular between 7 and 15, in particular between 8 and 14, in particular between 9 and 13, in particular between 10 and 12.

Afterwards, the signal is recognized or identified as ectopic signal if the signal belongs to at least one of two consecutive R-R intervals and if the two conditions explained in the following are fulfilled. In this context, it should be noted that a signal typically belongs to two different consecutive R-R intervals since the first R-R interval ends at the signal, wherein a consecutive second R-R interval starts at the same signal. However, in certain instances, it might also be possible that an individual cardiac signal only belongs to one R-R interval.

The following two conditions need to be met for recognizing a signal as ectopic signal. First, the first of the two consecutive R-R intervals needs to be at least 5% shorter than the average R-R interval. Second, a second of the two consecutive R-R intervals needs to be at least 5% longer than the average R-R interval. In this context, the second R-R interval occurs later than the first R-R interval in the electrocardiogram.

In order to achieve a particularly reliable recognition of ectopic signals, the first and the second of the two consecutive R-R intervals are discarded from calculating the average R-R interval if the signal is recognized as ectopic signal. Avoiding using of the two consecutive R-R intervals (also referred to as ectopic R-R intervals) for calculating the average R-R interval results in a more reliable average R-R interval and thus in a more reliable detection of subsequent ectopic signals.

In contrast to advanced computational methods used by many prior art ectopy detection algorithms, the presently described method can be performed with only very low power consumption so that it is particularly appropriate to be used in low-power implants.

In an embodiment, the method further comprises the steps explained in the following. A first deviation is calculated, wherein the first deviation is an average absolute deviation of the consecutive R-R intervals from the average R-R interval. In this context, the consecutive R-R intervals belong to the determinable number of latest consecutive R-R intervals used for calculating the average R-R interval. This first deviation is then multiplied with a factor being greater than one, resulting an increased first deviation. Furthermore, a second deviation is calculated. The second deviation is an absolute deviation of the successive R-R interval from the average R-R interval. "Successive R-R interval" means that this R-R interval succeeds the latest R-R interval of the determinable number of latest consecutive R-R intervals. The considered R-R interval is defined to be significantly shorter than the average R-R interval if a) the considered R-R interval is shorter than the average R-R interval and b) if the second deviation is greater than the increased first deviation. The considered R-R interval is defined to be significantly longer than the average R-R interval if a) the considered R-R interval is longer than the average R-R interval and b) if the second deviation is greater than the increased first deviation.

Thus, this embodiment does not rely on a comparison of consecutive R-R intervals nor on a calculation of a ratio between different R-R intervals. Rather, the reference point for deciding whether a considered R-R interval is a particularly long or a particularly short R-R interval is the average R-R interval. Thereby, the factor being greater than one determines a safety margin applied for classifying individual R-R intervals as ectopic R-R intervals. The bigger the factor, the less likely is the classification of individual R-R intervals as ectopic R-R intervals. Since this embodiment does not rely on or necessitates a static threshold, but rather enables use of a dynamic average R-R interval being updated continuously (due to use of always the actual latest R-R intervals for calculating the average R-R interval), the ectopy detection is continuously adapted to possible changes in the cardiac rhythm of the considered patient.

In an embodiment, the factor is a number between 1.05 and 5, in particular between 1.1 and 4.9, in particular between 1.2 and 4.8, in particular between 1.3 and 4.7, in particular between 1.4 and 4.6, in particular between 1.5 and 4.5, in particular between 1.6 and 4.4, in particular between 1.7 and 4.3, in particular between 1.8 and 4.2, in particular between 1.9 and 4.1, in particular between 2.0 and 4.0, in particular between 2.1 and 3.9, in particular between 2.2 and 3.8, in particular between 2.3 and 3.7, in particular between 2.4 and 3.6, in particular between 2.5 and 3.5, in particular between 2.6 and 3.4, in particular between 2.7 and 3.3, in particular between 2.8 and 3.2, in particular between 2.9 and 3.1, in particular between 3.0 and 3.1. A factor lying in a range between 1.5 and 2.5 is particularly appropriate for carrying out this embodiment.

In an embodiment, the first deviation is recalculated if the average R-R interval is updated. As outlined above, the R-R interval is typically regularly updated with each new incoming cardiac signal delimiting a further R-R interval. Thus, this recalculation of the first deviation is, in an embodiment, carried out continuously.

In an embodiment, the average R-R interval is recalculated with each newly detected R-R interval if this newly detected R-R interval is not discarded from calculating the average R-R interval. Thus, while R-R intervals comprising an ectopic cardiac signal are also in this embodiment discarded from calculating the average R-R interval, this embodiment guarantees for continuous update of the average R-R interval so that the average R-R interval is adapted to any changes in the cardiac rhythm.

As explained above, the first and the second of the two consecutive R-R intervals are discarded from calculating the average R-R interval in case that an ectopic signal has been recognized in either of the first or the second of the two consecutive R-R intervals. In an embodiment, also two R-R intervals preceding the first of the two consecutive R-R intervals are discarded from detection of the presence of atrial fibrillation.

In an embodiment, also the oldest R-R interval available for calculating the average R-R interval is discarded from detection of the presence of atrial fibrillation.

In an embodiment, the first of the two consecutive R-R intervals is considered to be significantly shorter than the average R-R interval if the first of the two consecutive R-R intervals is at least 5%, in particular between 5% and 100%, in particular between 10% and 90%, in particular between 20% and 80%, in particular between 30% and 70%, in particular between 40% and 60%, in particular 50% shorter than the average R-R interval.

In an embodiment, the second of the two consecutive R-R intervals is considered to be significantly longer than the average R-R interval if the second of the two consecutive R-R intervals is at least 5%, in particular between 5% and 500%, in particular between 10% and 490%, in particular between 20% and 480%, in particular between 30% and 470%, in particular between 40% and 460%, in particular between 50% and 450%, in particular between 60% and 440%, in particular between 70% and 430%, in particular between 80 % and 420%, in particular between 90% and 410%, in particular between 100% and 400%, in particular between 110% and 390%, in particular between 120% and 380%, in particular between 130% and 370%, in particular between 140% and 360%, in particular between 150% and 350%, in particular between 160% and 340%, in particular between 170% and 330%, in particular between 180% and 320%, in particular between 190% and 310%, in particular between 200% and 300%, in particular between 210% and 290 %, in particular between 220% and 280%, in particular between 230% and 270%, in particular between 240% and 260%, in particular between 250% an 260%, longer than the average R-R interval.

In an embodiment, a number of detected ectopic signals is counted. This counted number can then be used in other methods for making specific decisions, e.g., whether or not certain sections of the cardiac rhythm are to be used for the detection of atrial fibrillation or whether certain sections of an electrocardiogram are to be discarded.

In an aspect, the present invention relates to an implantable medical device for detecting an electrical signal of a human or animal heart. This implantable medical device comprises a processor, a memory unit, and a detection unit configured to detect electrical signals of a human or animal heart. In this context, the memory unit comprises a computer readable program that causes the processor to perform the following steps when executed on the processor.

In a first step, consecutive R-R intervals are detected or identified in an electrocardiogram.

Afterwards, an average R-R interval is calculated for a determinable number of latest consecutive R-R intervals.

Afterwards, the signal is recognized or identified as ectopic signal if the signal belongs to at least one of two consecutive R-R intervals and if the two conditions explained in the following are fulfilled.

The following two conditions need to be met for recognizing a signal as ectopic signal. First, the first of the two consecutive R-R intervals needs to be at least 5% shorter than the average R-R interval. Second, a second of the two consecutive R-R intervals needs to be at least 5% longer than the average R-R interval. In this context, the second R-R interval occurs later than the first R-R interval in the electrocardiogram.

In order to achieve a particularly reliable recognition of ectopic signals, the first and the second of the two consecutive R-R intervals are discarded from calculating the average R-R interval if the signal is recognized as ectopic signal. Avoiding using of the two consecutive R-R intervals (also referred to as ectopic R-R intervals) for calculating the average R-R interval results in a more reliable average R-R interval and thus in a more reliable detection of subsequent ectopic signals.

In an aspect, the present invention relates to a computer program product, in particular to a non-transitory computer program product, comprising computer readable code that causes a processor to perform the following steps when executed on the processor.

In a first step, consecutive R-R intervals are detected or identified in an electrocardiogram by a detection unit.

Afterwards, an average R-R interval is calculated for a determinable number of latest consecutive R-R intervals.

Afterwards, the signal is recognized or identified as ectopic signal if the signal belongs to at least one of two consecutive R-R intervals and if the two conditions explained in the following are fulfilled.

The following two conditions need to be met for recognizing a signal as ectopic signal. First, the first of the two consecutive R-R intervals needs to be significantly shorter than the average R-R interval. Second, a second of the two consecutive R-R intervals needs to be significantly longer than the average R-R interval. In this context, the second R-R interval occurs later than the first R-R interval in the electrocardiogram.

Finally, the first and the second of the two consecutive R-R intervals are discarded from calculating the average R-R interval if the signal is recognized as ectopic signal.

In an aspect, the present invention relates to a method for detecting atrial fibrillation of a human or animal heart, wherein the method comprises applying an atrial fibrillation detection algorithm (such as a common atrial fibrillation detection algorithm generally known per se to a person skilled in the art) and a method for detecting an ectopic signal in an electrocardiogram according to the preceding explanations. Then, any atrial fibrillation can be much more reliably detected since ectopic signals are more reliably detected than according to prior art methods.

In an embodiment, the method for detecting an ectopic signal in an electrocardiogram is only carried out for a programmable time period after beginning of the atrial fibrillation detection algorithm. In an embodiment, the programmable time period lies in a range between 30 seconds and 6 minutes, in particular between 1 minute and 5 minutes, in particular between 1.5 and 4.5 minutes, in particular between 2 and 4 minutes, in particular between 2.5 and 3.5 minutes, in particular 3 minutes.

According to an embodiment, the method for detecting an ectopic signal in an electrocardiogram is only carried out or for a programmable time period after the detection of an atrial fibrillation episode.

In an embodiment, a number of detected ectopic signals is counted, wherein an observed cardiac rhythm is considered being a rhythm devoid of atrial fibrillation if the number of detected ectopic signals exceeds a threshold. As an example, the observed cardiac rhythm is considered devoid of atrial fibrillation if 2 to 10 ectopic signals are detected within 10 to 25 intervals. In particular, the observed cardiac rhythm is considered devoid of atrial fibrillation if 4 ectopic signals are detected within 16 intervals.

According to an embodiment of the invention, an observed cardiac rhythm is considered being a rhythm devoid of atrial fibrillation if the number of detected ectopic signals plus a number of detected noise events exceeds a threshold.

All embodiments and variants of the different methods described herein can be combined in any desired way and can be transferred individually or in any arbitrary combination to the respective other method, to the computer program product and to the implantable medical device. Likewise, all variants and embodiments of the described implantable medical device can be combined in any desired way and can be transferred individually or in any arbitrary combination to either of the methods or to the described computer program product. Finally, all variants and embodiments described with respect to the computer program product can be combined in any desired way and can be transferred either individually or in any arbitrary combination to any of the described methods and to the implantable medical device.

Further details of aspects of the present invention will be explained with respect to exemplary embodiments and accompanying Figures. In the Figures:
- Fig. 1: is an exemplary section of an electrocardiogram comprising a premature ventricular contraction (PVC);
- Fig. 2: shows an example of a buffer of intervals used by an algorithm for detecting atrial fibrillation;
- Fig. 3: shows a schematic flowchart of an exemplary embodiment of a method for detecting an ectopic signal in an electrocardiogram;
- Fig. 4: shows a schematic flowchart of a method of atrial fibrillation detection with integrated ectopy detection;
- Fig. 5: shows a schematic flowchart of a method of ectopy detection threshold calculations;
- Fig. 6: shows a first exemplary electrocardiogram and a first ectopy detection result plot; and
- Fig. 7: shows a second exemplary electrocardiogram and a second ectopy detection result plot.

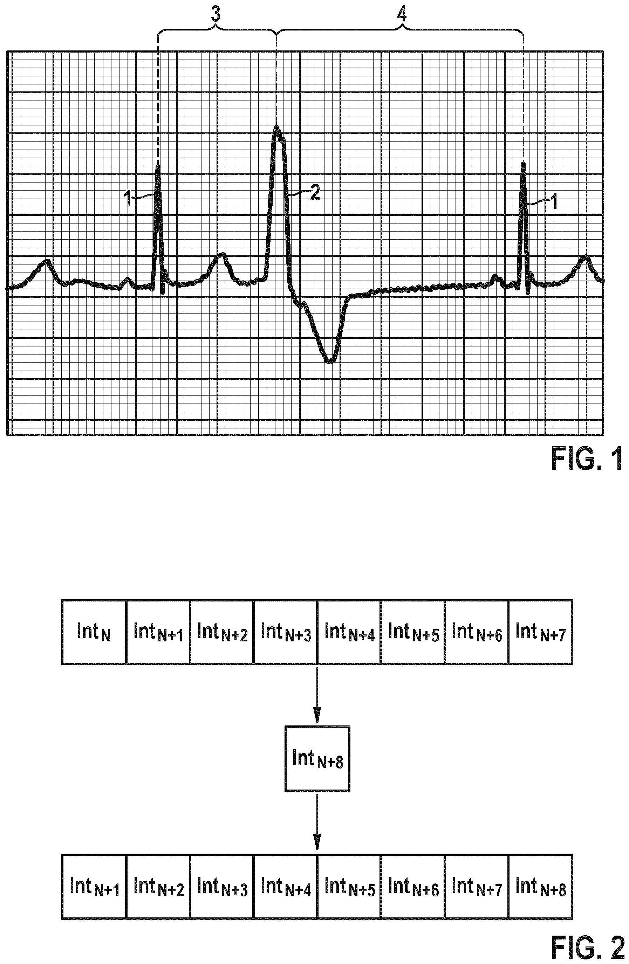

Figure 1 shows an exemplary section of an electrocardiogram. Two regular ventricular depolarizations 1 can be seen in the first third and in the last third of this electrocardiogram. In between, a premature ventricular contraction (PVC) 2 is visible. A first R-R interval 3 extends from the first normal ventricular depolarization 1 to the PVC 2.

This first R-R interval 3 is a short interval. A second R-R interval 4 extends from the PVC 2 to the second regular ventricular depolarization 1. This second R-R interval 4 is a compensatory pause interval being longer than usual R-R intervals.

The presence of the short R-R interval 3 followed by the long R-R interval 4 is indicative for an ectopy caused by an ectopic beat in form of the PVC 2.

The first normal ventricular depolarization 1 belongs both to the short R-R interval 3 as well as to the preceding R-R interval. The PVC 2 belongs both to the short R-R interval 3 as well as to the long R-R interval 4. The second normal ventricular depolarization 1 belongs both to the long R-R interval 4 as well as to the next succeeding R-R interval.

Figure 2 generally shows how individual R-R intervals of an electrocardiogram are stored in a buffer of an implantable medical device for sensing or detecting electrical signals of a human or animal heart. This general proceeding does not only apply for methods for detecting an ectopic signal in an electrocardiogram, but more broadly also for other signal processing algorithms like atrial fibrillation (AF) detection algorithms.

The top of Figure 2 shows a row representing a buffer of stored R-R intervals, wherein Int_{N} is the oldest interval and the intervals Int_{N+1}, Int_{N+2}, Int_{N+3}, Int_{N+4}, Int_{N+5}, Int_{N+6} are progressively newer. Int_{N+7} is the newest interval. This row of intervals can be considered as a number of latest consecutive R-R intervals, wherein the number is - in the exemplary embodiment depicted in Figure 2 - eight.

If a novel R-R interval Int_{N+8} is measured, this novel interval is added to the row of intervals, wherein the oldest interval Int_{N} is deleted. Consequently, now Int_{N+1} is the oldest interval, wherein Int_{N+8} is the newest interval. Still then, the buffer comprises eight latest consecutive R-R intervals Int_{N+1} to Int_{N+8}.

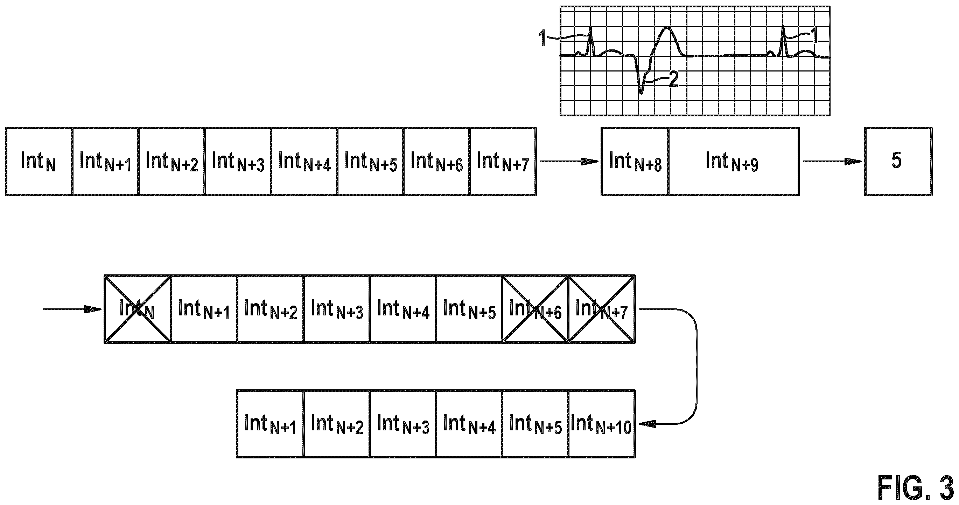

Figure 3 exemplarily shows an embodiment of a method for detecting an ectopic signal in an electrocardiogram. Starting with eight R-R intervals Int_{N} to Int_{N+7} (as explained with respect to Figure 2), two further R-R intervals Int_{N+8} and Int_{N+9} are detected. The R-R interval Int_{N+8} is a short R-R interval reaching from a regular ventricular depolarization 1 to a premature ventricular contraction (PVC) 2. Here and in all following Figures, the same numeral references for the same or similar elements are used.

The R-R interval Int_{N+9} is a longer interval reaching from the PVC 2 to a second regular ventricular depolarization 1. Consequently, an R-R interval being shorter than the average R-R intervals and a consecutive R-R interval being longer than the average R-R interval would be added to the row of R-R intervals Int_{N} to Int_{N+7}. This specific pattern of the short R-R interval Int_{N+8} and the long interval Int_{N+9} results in a detection 5 of an ectopic signal in the electrocardiogram. As a consequence, these two consecutive R-R intervals Int_{N+8} and Int_{N+9} are discarded from a buffer for detection of the presence of atrial fibrillation. Furthermore, the two intervals Int_{N+6} and Int_{N+7} preceding these two consecutive intervals are also discarded from the buffer for detection of the presence of atrial fibrillation. Finally, the oldest R-R interval Int_{N} is also discarded from the buffer for detection of the presence of atrial fibrillation.

After having detected the next regular R-R interval Int_{N+10}, a reduced row of R-R intervals comprising Int_{N+1}, Int_{N+2}, Int_{N+3}, Int_{N+4}, Int_{N+5}, and Int_{N+10} results. Then, only these intervals are used for calculating the average R-R interval. Intrr+s and Int_{N+10} are now considered and treated like consecutive R-R intervals in the interval buffer.

Discarding the first short R-R interval Int_{N+8} and the second long R-R interval i Int_{N+9} as well as the two R-R intervals Int_{N+6} and Int_{N+7} preceding the two short and long R-R intervals results in a significant more accurate calculation of the average R-R interval. Discarding also the oldest R-R interval Int_{N} from the calculation of the average R-R interval further enhances reliability, but is, in other embodiments of the method for detecting an ectopic signal in an electrocardiogram - not implemented.

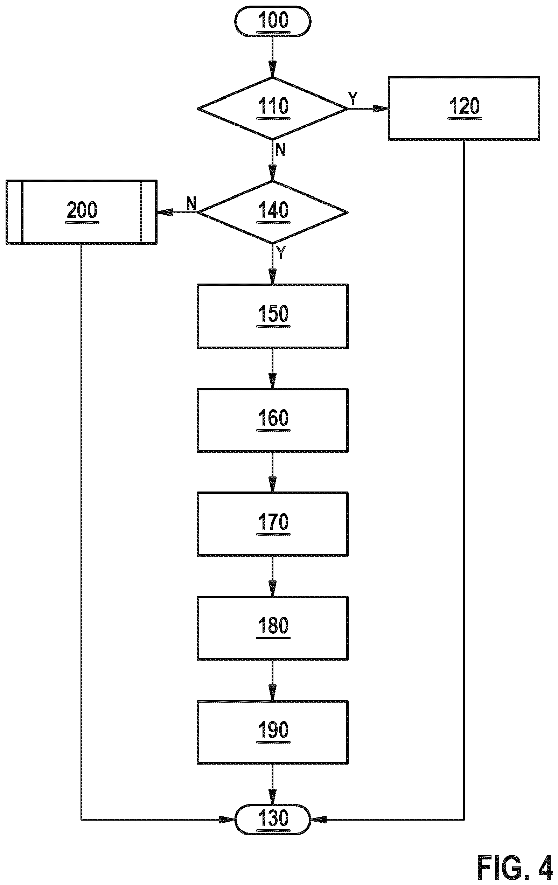

Figure 4 shows a schematic flowchart of a method of atrial fibrillation detection with integrated ectopy detection.

A QRS complex is detected in an electrocardiogram in a first method step 100 prior to detection of atrial fibrillation. Then, the method for detecting an ectopic signal in an electrocardiogram checks in a second step 110 if a parameter called "IgnoreOneInterval" is set to true. If this is the case (Y), this parameter is set to false in further step 120. Afterwards, the method for atrial fibrillation detection is continued in a regular method step 130. The parameter "IgnoreOneInterval" is said to true if an ectopic signal has been detected in the preceding method step, namely by the presence of an R-R interval being shorter than a short threshold followed by an R-R interval being longer than a long threshold. In such a case, normal atrial fibrillation detection 130 is to be applied rather than checking if another ectopic signal is to be detected in the electrocardiogram.

If, however, the parameter "IgnoreOneInterval" is not set to true (N), ectopy detection takes place. In a further method step 140, it is checked if the older of two consecutive R-R intervals was shorter than or equal to a short threshold and if at the same time the newer of two consecutive R-R intervals was longer than or equal to a long threshold. If this is true (Y), the result of method step 140 is a detected ectopy 150. Following ectopy detection, the ventricular signal that concludes a short interval is changed to a noise event (ventricular noise) in the next method step 160 for the atrial fibrillation algorithm. In addition, the R-R interval count is decremented twice in the next method step 170. Afterwards, the two newest intervals are removed from the R-R interval buffer in the next method step 180. According to an embodiment of the present invention, the ectopy detection is considered as ventricular noise by the atrial fibrillation detection algorithm, these ectopy detections lead to atrial fibrillation detection termination during the noise window that starts at the atrial fibrillation detection. According to an alternative embodiment, an ectopy detection leads to termination of an atrial fibrillation detection during the ectopy window, regardless of whether the noise window is simultaneously ongoing. When the interval buffer is full, then the noise count that leads to atrial fibrillation detection termination is cleared. Finally, in the next method step 190, the parameter "IgnoreOneInterval" is set to true so that the next interval is not allowed to change the ectopy detection thresholds. In any case, now a regular atrial fibrillation detection algorithm 130 is carried out.

If in the method step 140 the older R-R interval was not shorter than the short threshold and/or the newer R-R interval was not longer than the long threshold (N), then an update 200 of the short and long thresholds for ectopy detection is carried out. After this update 200, a regular atrial fibrillation detection algorithm 130 is carried out.

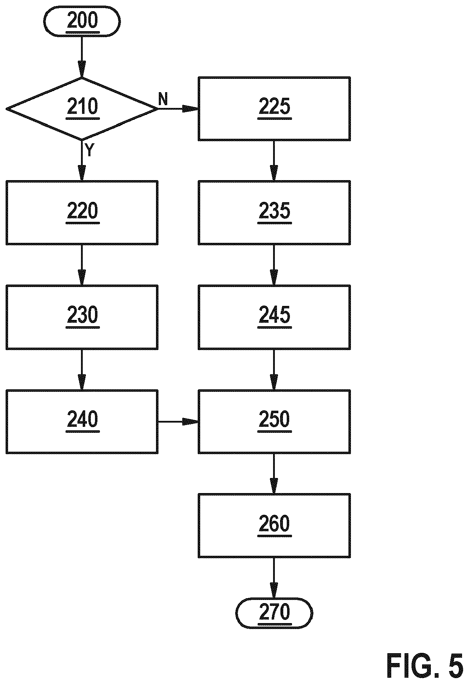

Figure 5 shows an exemplary embodiment of the update process 200 of Figure 4.

In a first step 210 of this update process, it is checked if the parameter "BuffLen" is equal to 1. This parameter represents the size of the average used for the mean R-R interval and the mean deviation from the average R-R interval (this deviation parameter is also called "intDeltaX"). BuffLen is an integer that will never be smaller than 1.

If BuffLen is 1 (Y), then the average R-R interval is the previous R-R interval, and the mean absolute deviation from the average is the difference between the old R-R interval and the previous R-R interval (intDeltaX = | (aveInt - oldInt) |) (step 220).

The variable "intDelta" is calculated as the maximum of "intDeltaX" times "DeltaLim" and the average interval times the parameter "LoIntLim" (intDelta = MAX(intDeltaX * DeltaLim, aveInt * LoIntLim) (step 230).

Finally, the actual average R-R interval (aveInt) is equal to the previous old R-R interval (oldInt) (aveInt = oldInt) (step 240). This method then continues with method step 250 (see below).

If BuffLen is greater than 1 (N), the average interval is updated with an exponential moving average (EMA) filter (aveInt = aveInt * (BuffLen - 1)BuffLen + (1/BuffLen) * oldInt) (step 225). In addition, the average interval difference from the mean (intDeltaX) is updated using an EMA filter (intDeltaX = intDeltaX * (BuffLen - 1)BuffLen + (1/BuffLen) * | (aveInt - oldInt) |) (step 235). Once intDeltaX is calculated, intDelta is updated. The variable "intDelta" is also in this branch of the method the maximum of "intDeltaX" times "DeltaLim" and the average interval times the parameter "LoIntLim" (intDelta = MAX(intDeltaX * DeltaLim, aveInt * LoIntLim) (step 245).

The variable "intDelta" is then subtracted from the variable "aveInt" to create the short threshold ectopy detection limit for the next ectopy test (short threshold = aveInt - intDelta) (step 250). Likewise, the variable "intDelta" is then added to the variable "aveInt" to create the long threshold ectopy detection limit for the next ectopy test (long threshold = aveInt + intDelta) (step 260).

Finally, an exit 270 of the update process is reached.

An aspect of the presently described method for detecting an ectopic signal in an electrocardiogram is that the ectopy detection is less sensitive as the beat-to-beat interval variability increases. Thus, it is harder to falsely detect ectopies during atrial fibrillation or during other irregular cardiac rhythms having a large beat-to-beat interval variability.

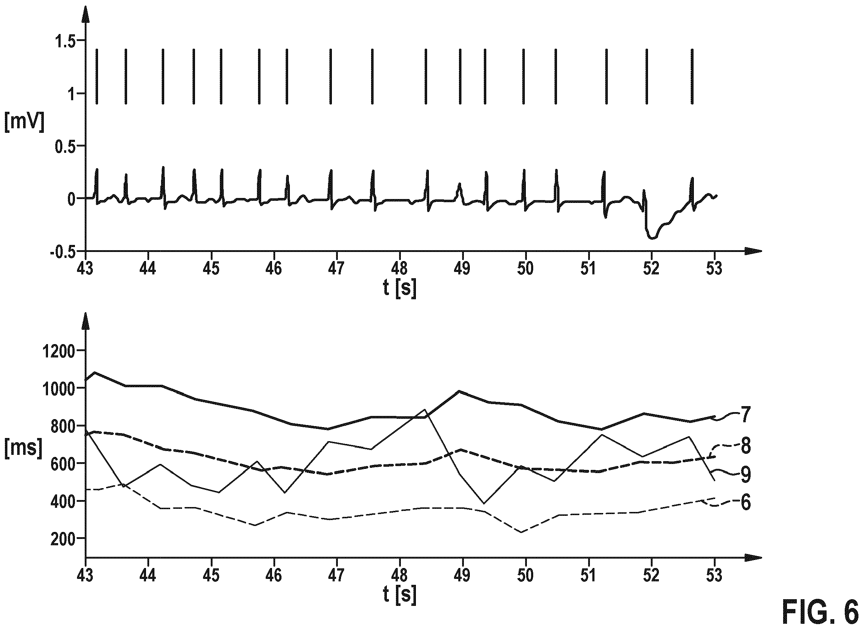

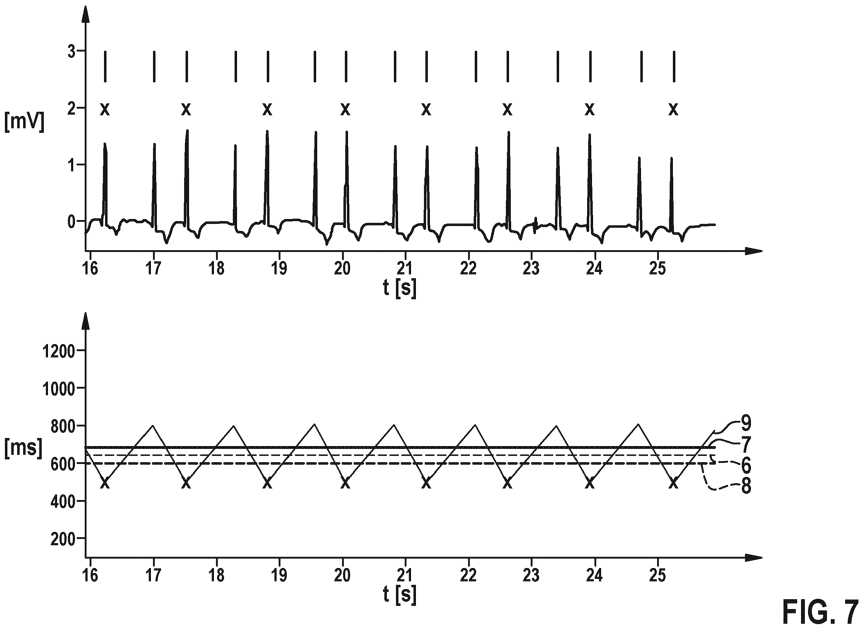

Figures 6 and 7 exemplarily show the behavior of the novel method for detecting an ectopic signal in an electrocardiogram making reference to an electrocardiogram and its evaluation as measured by an implantable medical device for detecting electrical signals of a human or animal heart.

Both in Figure 6 and in Figure 7, the upper panel shows an electrocardiogram with signals in mV over the time in seconds. Ventricular signals in the electrocardiogram are marked with vertical lines in the top of the top panels of Figures 6 and 7. The bottom plots of Figures 6 and 7 show an ectopy algorithm average interval 6, an upper ectopy detection threshold 7, a lower ectopy detection threshold 8, and actual R-R intervals 9. The ectopy algorithm average interval 6 forms the basis for calculating the upper ectopy detection threshold 7 (longer threshold) and the lower ectopy detection threshold 8 (short threshold).

In case of atrial fibrillation (cf. Figure 6), the actual R-R intervals 9 are highly volatile. Consequently, a wide corridor is formed by the upper ectopy detection threshold 7 and the lower ectopy detection threshold 8. Consequently, no ectopy is detected during this cardiac rhythm state.

In contrast, Figure 7 shows a typical sinus rhythm of a heart resulting in a narrow corridor between the upper ectopy detection threshold 7 and the lower ectopy detection threshold 8. As a result, a plurality of detected cardiac signals is classified as ectopic signals, each resulting from an ectopic beat (marked with an X in the upper panel of Figure 7). In this case, due to a lack of continuous updates of the short and long thresholds, the depicted algorithm is prone to false detections of ectopic beats. Thus, using the continuous update of the short and long thresholds for detecting ectopic signals according to the presently described invention, the method is less prone to falsely detect ectopic signals in case of atrial fibrillation than methods known from prior art. At the same time, ectopic signals can be reliably detected during times of stable cardiac sinus rhythm.

## Claims

1. A method for detecting an ectopic signal in an electrocardiogram, the ectopic signal being caused by an ectopic cardiac beat, the method comprising the following steps that are performed by a processor of an implantable medical device when a computer-readable program comprised in a memory of the implantable medical device is executed on the processor:
detecting, with a detection unit of the implantable medical device, consecutive R-R intervals in an electrocardiogram;
calculating an average R-R interval for a determinable number of latest R-R intervals;
recognizing a signal as ectopic signal if the signal belongs to at least one of two consecutive R-R intervals, wherein
i) a first of the two consecutive R-R intervals is at least 5% shorter than the average R-R interval; and
ii) a second of the two consecutive R-R intervals is at least 5% longer than the average R-R interval, wherein the second R-R interval occurs later than the first R-R interval;
wherein the first and the second of the two consecutive R-R intervals are discarded from calculating the average R-R interval, if the signal is recognized as ectopic signal.

2. The method according to claim 1, wherein the method further comprises the following steps: calculating a first deviation, the first deviation being an average absolute deviation of the consecutive R-R intervals of the determinable number of latest consecutive R-R intervals used for calculating the average R-R interval from the average R-R interval; multiplying the first deviation with a factor being greater than 1 to obtain an increased first deviation; calculating a second deviation, the second deviation being an absolute deviation of a successive R-R interval from the average R-R interval; defining the R-R interval as significantly shorter than the average R-R interval if the R-R interval is shorter than the average R-R interval and if the second deviation is greater than the increased first deviation, or defining the R-R interval as significantly longer than the average R-R interval if the R-R interval is longer than the average R-R interval and if the second deviation is greater than the increased first deviation.

3. The method according to claim 2, wherein the factor is a number between 1.05 and 5.

4. The method according to claim 2 or 3, wherein the first deviation is recalculated if the average R-R interval is updated.

5. The method according to one of the preceding claims, wherein the average R-R interval is recalculated with each newly detected R-R interval if this newly detected R-R interval is not discarded from calculating the average R-R interval.

6. The method according to one of the preceding claims, wherein, if the first and the second of the two consecutive R-R intervals are discarded from calculating the average R-R interval.

7. The method according to one of the preceding claims, wherein the first of the two consecutive R-R intervals is considered to be significantly shorter than the average R-R interval if the first of the two consecutive R-R intervals is at least 5% shorter than the average R-R interval.

8. The method according to one of the preceding claims, wherein the second of the two consecutive R-R intervals is considered to be significantly longer than the average R-R interval if the second of the two consecutive R-R intervals is at least 5% longer than the average R-R interval.

9. The method according to one of the preceding claims, wherein a number of detected ectopic signals is counted.

10. An implantable medical device for detecting an electrical signal of a human or animal heart, the implantable medical device comprising a processor, a memory unit, and a detection unit configured to detect an electrical signal of a human or animal heart, wherein the memory unit comprises a computer-readable program that causes the processor to perform the following steps when executed on the processor:
detecting, with the detection unit, consecutive R-R intervals in an electrocardiogram;
calculating an average R-R interval for a determinable number of latest consecutive R-R intervals;
recognizing a signal as ectopic signal if the signal belongs to at least one of two consecutive R-R intervals, wherein
i) a first of the two consecutive R-R intervals is at least 5% shorter than the average R-R interval; and
ii) a second of the two consecutive R-R intervals is at least 5% longer than the average R-R interval, wherein the second R-R interval occurs later than the first R-R interval;
wherein the first and the second of the two consecutive R-R intervals are discarded from calculating the average R-R interval, if the signal is recognized as ectopic signal.

## Patentansprüche

1. Verfahren zum Erkennen eines ektopischen Signals in einem Elektrokardiogramm, wobei das ektopische Signal durch einen ektopischen Herzschlag verursacht wird, wobei das Verfahren die folgenden Schritte umfasst, die von einem Prozessor einer implantierbaren medizinischen Vorrichtung durchgeführt werden, wenn ein computerlesbares Programm, das in einem Speicher der implantierbaren medizinischen Vorrichtung enthalten ist, auf dem Prozessor ausgeführt wird:
Erfassen von aufeinanderfolgenden R-R-Intervallen in einem Elektrokardiogramm mit einer Erfassungseinheit der implantierbaren medizinischen Vorrichtung;
Berechnung eines durchschnittlichen R-R-Intervalls für eine bestimmbare Anzahl von letzten R-R-Intervallen;
Erkennen eines Signals als ektopisches Signal, wenn das Signal zu mindestens einem von zwei aufeinanderfolgenden R-R-Intervallen gehört, wobei
i) ein erstes der beiden aufeinanderfolgenden R-R-Intervalle ist mindestens 5 % kürzer als das durchschnittliche R-R-Intervall; und
ii) ein zweites der beiden aufeinanderfolgenden R-R-Intervalle ist mindestens 5 % länger als das durchschnittliche R-R-Intervall, wobei das zweite R-R-Intervall später als das erste R-R-Intervall auftritt;
wobei das erste und das zweite der beiden aufeinanderfolgenden R-R-Intervalle bei der Berechnung des durchschnittlichen R-R-Intervalls ausgeschlossen werden, wenn das Signal als ektopisches Signal erkannt wird.

2. Verfahren nach Anspruch 1, wobei das Verfahren ferner die folgenden Schritte umfasst: Berechnen einer ersten Abweichung, wobei die erste Abweichung eine durchschnittliche absolute Abweichung der aufeinanderfolgenden R-R-Intervalle der bestimmbaren Anzahl der letzten aufeinanderfolgenden R-R-Intervalle, die zur Berechnung des durchschnittlichen R-R-Intervalls verwendet werden, von dem durchschnittlichen R-R-Intervall ist; Multiplizieren der ersten Abweichung mit einem Faktor, der größer als 1 ist, um eine vergrößerte erste Abweichung zu erhalten; Berechnen einer zweiten Abweichung, wobei die zweite Abweichung eine absolute Abweichung eines aufeinanderfolgenden R-R-Intervalls von dem durchschnittlichen R-R-Intervall ist; Definieren des R-R-Intervalls als signifikant kürzer als das durchschnittliche R-R-Intervall, wenn das R-R-Intervall kürzer als das durchschnittliche R-R-Intervall ist und wenn die zweite Abweichung größer als die erhöhte erste Abweichung ist, oder Definieren des R-R-Intervalls als signifikant länger als das durchschnittliche R-R-Intervall, wenn das R-R-Intervall länger als das durchschnittliche R-R-Intervall ist und wenn die zweite Abweichung größer als die erhöhte erste Abweichung ist.

3. Verfahren nach Anspruch 2, wobei der Faktor eine Zahl zwischen 1,05 und 5 ist.

4. Verfahren nach Anspruch 2 oder 3, wobei die erste Abweichung neu berechnet wird, wenn das durchschnittliche R-R-Intervall aktualisiert wird.

5. Verfahren nach einem der vorhergehenden Ansprüche, wobei das durchschnittliche R-R-Intervall mit jedem neu detektierten R-R-Intervall neu berechnet wird, wenn dieses neu detektierte R-R-Intervall bei der Berechnung des durchschnittlichen R-R-Intervalls nicht ausgeschlossen wird.

6. Verfahren nach einem der vorhergehenden Ansprüche, wobei, wenn das erste und das zweite der beiden aufeinanderfolgenden R-R-Intervalle bei der Berechnung des durchschnittlichen R-R-Intervalls ausgeschlossen werden.

7. Verfahren nach einem der vorhergehenden Ansprüche, wobei das erste der beiden aufeinanderfolgenden R-R-Intervalle als signifikant kürzer als das durchschnittliche R-R-Intervall angesehen wird, wenn das erste der beiden aufeinanderfolgenden R-R-Intervalle mindestens 5% kürzer als das durchschnittliche R-R-Intervall ist.

8. Verfahren nach einem der vorhergehenden Ansprüche, wobei das zweite der beiden aufeinanderfolgenden R-R-Intervalle als signifikant länger als das durchschnittliche R-R-Intervall angesehen wird, wenn das zweite der beiden aufeinanderfolgenden R-R-Intervalle mindestens 5% länger als das durchschnittliche R-R-Intervall ist.

9. Verfahren nach einem der vorhergehenden Ansprüche, wobei die Anzahl der erkannten ektopischen Signale gezählt wird.

10. Implantierbare medizinische Vorrichtung zum Erfassen eines elektrischen Signals eines menschlichen oder tierischen Herzens, wobei die implantierbare medizinische Vorrichtung einen Prozessor, eine Speichereinheit und eine Erfassungseinheit umfasst, die so konfiguriert ist, dass sie ein elektrisches Signal eines menschlichen oder tierischen Herzens erfasst, wobei die Speichereinheit ein computerlesbares Programm umfasst, das den Prozessor veranlasst, die folgenden Schritte durchzuführen, wenn es auf dem Prozessor ausgeführt wird:
Erkennung aufeinanderfolgender R-R-Intervalle in einem Elektrokardiogramm mit der Erfassungseinheit;
Berechnung eines durchschnittlichen R-R-Intervalls für eine bestimmbare Anzahl von letzten aufeinanderfolgenden R-R-Intervallen;
Erkennen eines Signals als ektopisches Signal, wenn das Signal zu mindestens einem von zwei aufeinanderfolgenden R-R-Intervallen gehört, wobei
i) ein erstes der beiden aufeinanderfolgenden R-R-Intervalle mindestens 5 % kürzer ist als das durchschnittliche R-R-Intervall; und
ii) ein zweites der beiden aufeinanderfolgenden R-R-Intervalle ist mindestens 5 % länger als das durchschnittliche R-R-Intervall, wobei das zweite R-R-Intervall später als das erste R-R-Intervall auftritt;
wobei das erste und das zweite der beiden aufeinanderfolgenden R-R-Intervalle bei der Berechnung des durchschnittlichen R-R-Intervalls ausgeschlossen werden, wenn das Signal als ektopisches Signal erkannt wird.

## Revendications

1. Procédé de détection d'un signal ectopique dans un électrocardiogramme, le signal ectopique étant amené par un battement cardiaque ectopique, le procédé comprenant les étapes suivantes qui sont réalisées par un processeur d'un dispositif médical implantable lorsqu'un programme lisible par ordinateur compris dans une mémoire du dispositif médical implantable est exécuté sur le processeur :
détection, à l'aide d'une unité de détection du dispositif médical implantable, d'intervalles R-R consécutifs dans un électrocardiogramme ;
calcul d'un intervalle R-R moyen pour un nombre déterminable d'intervalles R-R les plus tardifs ;
reconnaissance d'un signal comme signal ectopique si le signal appartient à au moins un parmi deux intervalles R-R consécutifs, dans lequel
i) un premier parmi les deux intervalles R-R consécutifs est plus court d'au moins 5 % que l'intervalle R-R moyen ; et
ii) un second parmi les deux intervalles R-R consécutifs est plus long d'au moins 5 % que l'intervalle R-R moyen, dans lequel le second intervalle R-R survient plus tard que le premier intervalle R-R ;
dans lequel le premier et le second des deux intervalles R-R consécutifs sont rejetés du calcul de l'intervalle R-R moyen, si le signal est reconnu comme signal ectopique.

2. Procédé selon la revendication 1, dans lequel le procédé comprend en outre les étapes suivantes : calcul d'un premier écart, le premier écart étant un écart absolu moyen des intervalles R-R consécutifs du nombre déterminable d'intervalles R-R consécutifs les plus tardifs utilisé pour calculer l'intervalle R-R moyen à partir de l'intervalle R-R moyen ; multiplication du premier écart par un facteur supérieur à 1 pour obtenir un premier écart augmenté ; calcul d'un second écart, le second écart étant un écart absolu d'un intervalle R-R successif à partir de l'intervalle R-R moyen ; définition de l'intervalle R-R comme significativement plus court que l'intervalle R-R moyen si l'intervalle R-R est plus court que l'intervalle R-R moyen et si le second écart est supérieur au premier écart augmenté, ou définition de l'intervalle R-R comme significativement plus long que l'intervalle R-R moyen si l'intervalle R-R est plus long que l'intervalle R-R moyen et si le second écart est supérieur au premier écart augmenté.

3. Procédé selon la revendication 2, dans lequel le facteur est un nombre entre 1,05 et 5.

4. Procédé selon la revendication 2 ou 3, dans lequel le premier écart est recalculé si l'intervalle R-R moyen est actualisé.

5. Procédé selon l'une des revendications précédentes, dans lequel l'intervalle R-R moyen est recalculé avec chaque intervalle R-R nouvellement détecté si cet intervalle R-R nouvellement détecté n'est pas rejeté du calcul de l'intervalle R-R moyen.

6. Procédé selon l'une des revendications précédentes, dans lequel, si le premier et le second des deux intervalles R-R consécutifs sont rejetés du calcul de l'intervalle R-R moyen.

7. Procédé selon l'une des revendications précédentes, dans lequel le premier des deux intervalles R-R consécutifs est considéré comme significativement plus court que l'intervalle R-R moyen si le premier des deux intervalles R-R consécutifs est plus court d'au moins 5 % que l'intervalle R-R moyen.

8. Procédé selon l'une des revendications précédentes, dans lequel le second des deux intervalles R-R consécutifs est considéré comme significativement plus long que l'intervalle R-R moyen si le second des deux intervalles R-R consécutifs est plus long d'au moins 5 % que l'intervalle R-R moyen.

9. Procédé selon l'une des revendications précédentes, dans lequel un nombre de signaux ectopiques détectés est décompté.

10. Dispositif médical implantable destiné à la détection d'un signal électrique d'un coeur humain ou animal, le dispositif médical implantable comprenant un processeur, une unité de mémoire et une unité de détection conçue pour détecter un signal électrique d'un coeur humain ou animal, dans lequel l'unité de mémoire comprend un programme lisible par ordinateur qui amène le processeur à réaliser les étapes suivantes lorsqu'il est exécuté sur le processeur :
détection, à l'aide de l'unité de détection, d'intervalles R-R consécutifs dans un électrocardiogramme ;
calcul d'un intervalle R-R moyen pour un nombre déterminable d'intervalles R-R consécutifs les plus tardifs ;
reconnaissance d'un signal comme signal ectopique si le signal appartient à au moins un parmi deux intervalles R-R consécutifs, dans lequel
i) un premier parmi les deux intervalles R-R consécutifs est plus court d'au moins 5 % que l'intervalle R-R moyen ; et
ii) un second parmi les deux intervalles R-R consécutifs est plus long d'au moins 5 % que l'intervalle R-R moyen, dans lequel le second intervalle R-R survient plus tard que le premier intervalle R-R ;
dans lequel le premier et le second des deux intervalles R-R consécutifs sont rejetés du calcul de l'intervalle R-R moyen, si le signal est reconnu comme signal ectopique.
